# EUROPEAN PATENT SPECIFICATION

(11) **EP 3 091 949 B1**
(45) Date of publication and mention of the grant of the patent: **03.04.2019**
(21) Application number: 14881612.7
(22) Date of filing: 03.10.2014
(51) Int. Cl.: A61F 7/12, A61F 7/00, A61F 7/02, F25B 39/02, F28F 3/12, F28D 9/00

(54) **PATIENT HEAT EXCHANGE SYSTEM WITH TRANSPARENT WALL FOR VIEWING CIRCULATING REFRIGERANT**
PATIENTENWÄRMEAUSTAUSCHSYSTEM MIT TRANSPARENTER WAND ZUM BETRACHTEN EINES ZIRKULIERENDEN KÄLTEMITTELS
SYSTÈME D'ÉCHANGE THERMIQUE POUR PATIENT POURVU D'UNE PAROI TRANSPARENTE PERMETTANT DE VOIR LA CIRCULATION D'UN FLUIDE FRIGORIGÈNE

(30) Priority: 07.02.2014 US 201414175545; 13.05.2014 US 201414276202
(43) Date of publication of application: 16.11.2016
(60) Divisional application: 19157571.1
(73) Proprietor: ZOLL Circulation, Inc., San Jose, CA 95131 (US)
(72) Inventor: DABROWIAK, Jeremy Thomas, Redwood City, California 94065 (US); PETERSON, Eric, Pacifica, California 94044 (US)
(74) Representative: Potter Clarkson
(86) International application number: PCT/US2014/059029
(87) International publication number: WO 2015/119671

(56) References cited:
- WO-A1-95/03680
- US-A- 3 140 716
- US-A- 5 263 925
- US-A- 5 269 749
- US-A- 5 403 281
- US-A1- 2003 114 795
- US-A1- 2008 262 409
- US-A1- 2008 262 409
- US-A1- 2009 247 963
- US-B1- 6 673 098
- US-B1- 6 878 156
- US-B2- 8 226 605

## Description

### I. FIELD OF THE INVENTION

The present application relates generally to patient heat exchange systems with a transparent wall for viewing circulating refrigerant.

### II. BACKGROUND OF THE INVENTION

Patient temperature control systems have been introduced to prevent fever in patients in the neuro ICO due to suffering from sub-arachnoid hemorrhage or other neurologic malady such as stroke. Also, such systems have been used to induce mild or moderate hypothermia to improve the outcomes of patients suffering from such maladies as stroke, cardiac arrest, myocardial infarction, traumatic brain injury, and high intracranial pressure. Examples of intravascular heat exchange catheter are disclosed in U.S. Patent Nos. 6,419,643, 6,416,533, 6,409,747, 6,405,080, 6,393,320, 6,368,304, 6,338,727, 6,299,599, 6,290,717, 6,287,326, 6,165,207, 6,149,670, 6,146,411, 6,126,684, 6306,161, 6,264,679, 6,231,594, 6,149,676, 6,149,673, 6,110,168, 5,989,238, 5,879,329, 5,837,003, 6,383,210, 6,379,378, 6,364,899, 6,325,818, 6,312,452, 6,261,312, 6,254,626, 6,251,130, 6,251,129, 6,245,095, 6,238,428, 6,235,048, 6,231,595, 6,224,624, 6,149,677, 6,096,068, 6,042,559.

External, patient temperature control systems may be used. Such systems are disclosed in U.S. Patent Nos. 6,827,728, 6,818,012, 6,802,855, 6,799,063, 6,764,391, 6,692,518, 6,669,715, 6,660,027, 6,648,905, 6,645,232, 6,620,187, 6,461,379, 6,375,674, 6,197,045, and 6,188,930 (collectively, "the external pad patents") US 6878156 describes, in one example, a cooler for heat exchange catheters, having two heat exchange plates and a heat exchange element disposable in the slot of a housing and sandwiched between the heat exchange plates to be in thermal contact therewith. The heat exchange element may be configured to have opposed flat flexible plastic layers that form a coolant space therebetween.

### SUMMARY OF THE INVENTION

The invention provides a heat exchange system according to claim 1 and a method of operating a heat exchange system according to claim 12. More generally, aspects of the present disclosure include a heat exchange system for exchanging heat with working fluid from an intravascular heat exchange catheter or an external heat exchange pad includes a refrigerant circuit configured for circulating refrigerant between a compressor and first and second cold plates between which a working fluid cassette is disposable. At least a first one of the cold plates has a transparent outer wall through which a person can view refrigerant flowing in the refrigerant circuit

In example embodiments a cassette slot is defined between the cold plates for receiving the fluid cassette. A distance between the cold plates (e.g., the width of the slot) can be less than forty mils (0.040") (1.02 mm). Plural fasteners may hold the outer wall onto metal structure of the first cold plate. The fasteners may be arranged in four rows, one respective row along each of four edges of the outer wall. The cold plates can be nearly square and can abut each other along left and right side walls. In examples, respective vertically elongated cassette frame receptacles are located immediately inboard of the respective side walls with the slot extending between the side walls and terminating at the receptacles, and the frame receptacles are wider than the slot. At least one cold plate may be formed with a serpentine passageway through which the refrigerant flows.

In another aspect, system includes two heat transfer plates parallel to each other and defining a slot between them configured for receiving a working fluid cassette through which working fluid flows to and from an intravascular catheter in a working fluid circuit. A refrigerant circuit supplies refrigerant to at least one of the plates to exchange heat therewith. The refrigerant circuit includes a compressor and may be the only fluid circuit in thermal contact with the working fluid circuit other than a bloodstream of a patient in which the catheter can be positioned. According to present principles, at least one of the heat transfer plates is at least partially transparent to permit viewing the refrigerant as it flows in the refrigerant circuit.

In another aspect, a method includes circulating refrigerant between a compressor and a cold plate, and enabling a person to view the refrigerant as it circulates.
The details of the present invention, both as to its structure and operation, can best be understood in reference to the accompanying drawings, in which like reference numerals refer to like parts, and in which:

### BRIEF DESCRIPTION OF THE DRAWINGS

Figure 1 is a schematic view of a non-limiting system in accordance with the present invention;
Figure 2 is a perspective view of an example working fluid cassette holder portion of a heat exchange system;
Figure 3 is another view of the transparent half of the cassette holder shown in Figure 2 omitting the fasteners and holes for clarity;
Figure 4 is a perspective view of art example working fluid cassette configured to engage the cassette holder shown in Figures 2 and 3; and
Figure 5 is a close up perspective view of the cassette shown in Figure 4, illustrating an inlet tube extending partially down into the stretched membrane chamber, it being understood that an opposed outlet tube may be similarly disposed on the opposite side of the cartridge and that both the inlet and outlet tubes may extend any length down their respective sides in the cassette.

### DETAILED DESCRIPTION OF THE PREFERRED EMBODIMENT

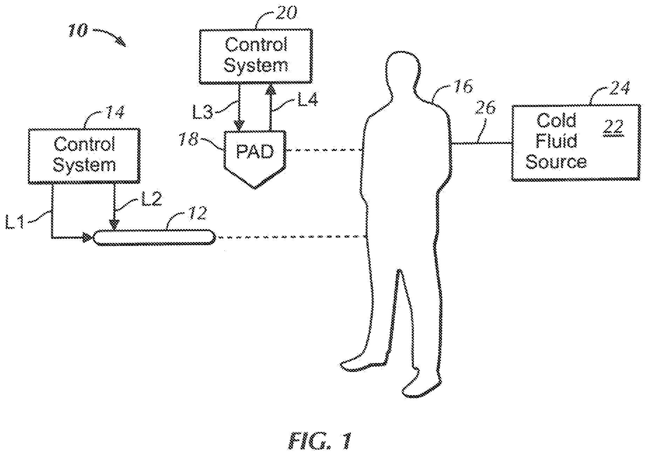

Referring initially to Figure 1, in accordance with present principles, a system 10 may include an intravascular heat exchange catheter 12 controlled by a control system 14 to induce control patient temperature, e.g., to prevent the patient 16 from becoming febrile or to induce therapeutic hypothermia in the patient 16. In the catheter, working fluid (also referred to as "coolant") such as but not limited to saline circulates (typically under the influence of a pump in the controller) in a closed loop from the control system 14, through a fluid supply line L1, through the catheter 12, and back to the system 14 through a fluid return line L2, such that no coolant enters the body. While certain preferred catheters are disclosed below, it is to be understood that other catheters can be used in accordance with present principles, including, without limitation, any of the catheters disclosed above or in the following U.S. USPN 5,486,208, 5,837,003, 6,110,168, 6,149,673, 6,149,676, 6,231,594, 6,264,679, 6,306,161, 6,235,048, 6,238,428, 6,245,095, 6,251,129, 6,251,130, 6,254,626, 6,261,312, 6,312,452, 6,325,818, 6,409,747, 6,368,304, 6,338,727, 6,299,599, 6,287,326, 6,126,684. The catheter 12 may be placed in the venous system, e.g., in the superior or inferior vena cava.

Instead of or in addition to the catheter 12, the system 10 may include one or more pads 18 that are positioned against the external skin of the patient 16 (only one pad 18 shown for clarity). The pad 18 may be, without limitation, any one of the pads disclosed in the external pad patents. The temperature of the pad 18 can be controlled by a pad control system 20 in accordance with principles set forth in the external pad patents to exchange heat with the patient 16, including to induce therapeutic mild or moderate hypothermia in the patient in response to the patient presenting with, e.g., cardiac arrest, myocardial infarction, stroke, high intracranial pressure, traumatic brain injury, or other malady the effects of which can be ameliorated by hypothermia. The pad 18 may receive working fluid from the system 20 through a fluid supply line L3, and return working fluid to the system 20 through a fluid return line L4. Note that in some embodiments, the systems 14, 20 are established in a single assembly.

To cool the patient while awaiting engagement of the catheter 12 and/or pad 18 with the patient, cold fluid 22 in a cold fluid source 24 may be injected into the patient and in particular into the patient's venous system through a pathway 26. Without limitation, the pathway 26 may an IV line, the source 24 may be an IV bag, and the fluid 22 may be chilled saline, e.g., saline at the freezing point or slightly warmer. Or, the source may be a syringe, and the saline can be injected directly into the bloodstream of the patient.

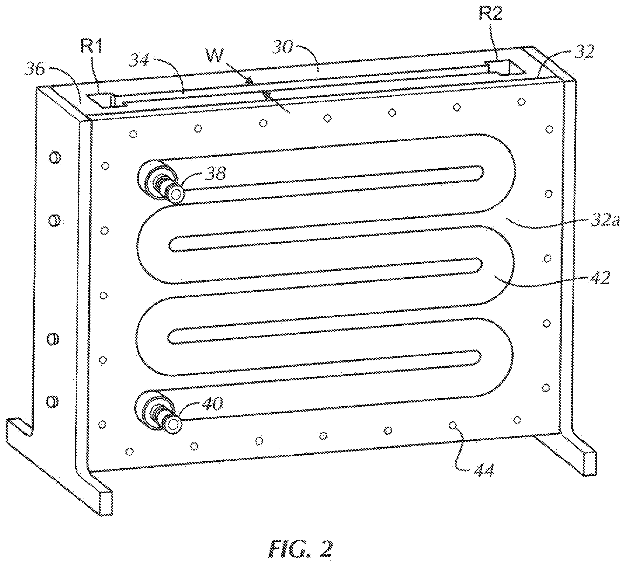

Now referring to Figure 2, a portion of either of the heat exchangers in the control systems 14, 20 is shown which includes at least two cold plates 30, 32 defining a cassette slot 34 between them, in one embodiment the width "W" of the slot 34 is less than forty mils (0.040") (1.02 mm), and may be between twenty nine mils and thirty one mils (0.029"-0.031") (0.74 - 0.79 mm) or may have a nominal slot width of 0.035" (0.89 mm). In a specific example the width "W" may be thirty mils (0.76 mm). In other embodiments, when a disposable heat exchange bag with serpentine channels is used, a larger gap between the cold plates may be used, e.g., 0.060" - 0.120" (1.52 - 3.05 mm) and more preferably 0.080" (2.03 mm) to promote pumping saline through the bag without excessive backpressure.

The cold plates 30, 32 may be made of metal with the exception noted below, and can be rectilinear as shown and indeed may be nearly square. The cold plates 30, 32 may abut each other along left and right side walls 36, with elongated vertical cassette frame receptacles R1 and R2 being located immediately inboard of the respective side walls 36 and with the slot 34 extending between the walls 36 and terminating at the receptacles R1, R2 as shown. The frame receptacles R1, R2 are wider than the slot 36.

In the example shown, refrigerant inlet and outlet tubes 38, 40 extend through at least one of the cold plates 32 to communicate refrigerant from a compressor into a refrigerant passageway in the cold plate. Each cold plate may have its own refrigerant inlet and outlet tubes, or, in the embodiment shown, only one cold plate may be formed with refrigerant inlet and outlet tubes and the other cold plate either thermally coupled to the cold plate in which the refrigerant flows and/or receiving refrigerant from the other cold plate through passageways formed through one or both of the side walls 36.

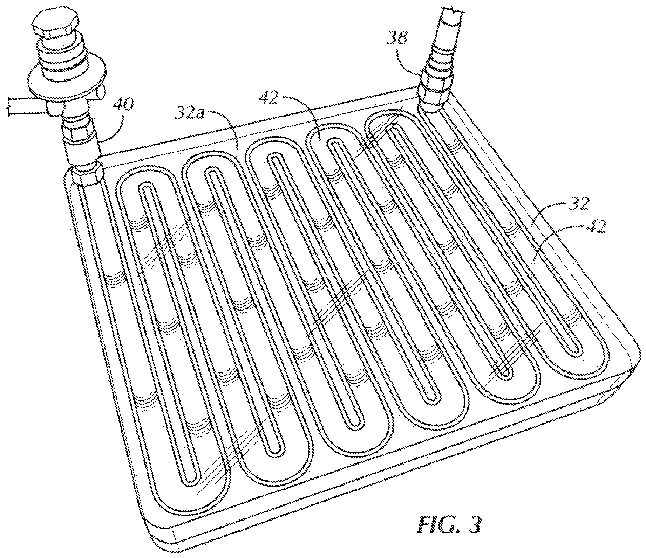

In the example shown in Figures 2 and 3, the outer wall of at least one of the cold plates is made at least partially (in the example shown, the entire wall is transparent) of transparent material such as hard plastic, e.g., plexiglass having a thickness of, e.g., one eighth of an inch (3.18 mm), so that a person can view refrigerant flowing inside the cold plate to verify proper operation. For example, the outer wall 32a of the cold plate 32 may be transparent as shown.

Thus, as shown in Figures 2 and 3, owing to the transparent outer wall 32a of the cold plate 32, the refrigerant, in this non-limiting example constrained to flow in a serpentine refrigerant passageway 42, can be viewed during operation by a person looking
at the outer wall 32a. The example refrigerant passageway that fluidly connects the refrigerant inlet 38 to the refrigerant outlet 40 may be serpentine-shaped as shown, or may be some other shape or pattern such as a herringbone pattern, a wave pattern, etc. Alternatively, parallel channel passages may be used. For instance, ten one-inch (25.4 mm) wide channels may be formed in parallel, thereby achieving a 10x10" (25.4 x 25.4 cm) surface area. Typically during operation the refrigerant bubbles as it transitions state while exchanging heat with the cassette and so the visual presentation to the viewer is of a flowing bubbling fluid.

Because the transparent outer wall 32a is exposed to fluid pressure from the inside, plural fasteners 44 (Figure 2) may be used to secure the outer wall 32a to the remaining metal structure of the cold plate 32. Note that Figure 3 does not show the fasteners for clarity, to better reveal the transparent view of the underlying refrigerant path.

The fasteners 44 shown in Figure 2 may be threaded fasteners such as bolts that are received in corresponding threaded receptacles with which they are registered in the metal structure of the cold plate 32. For fortified securement, Figure 2 shows that four rows of fasteners 44 may be used, a respective row of fasteners being arranged along each of the four edges of the wall 32a just inboard of the periphery of the wall. The spacing between adjacent fasteners may be, e.g., one half of an inch (12.7 mm).

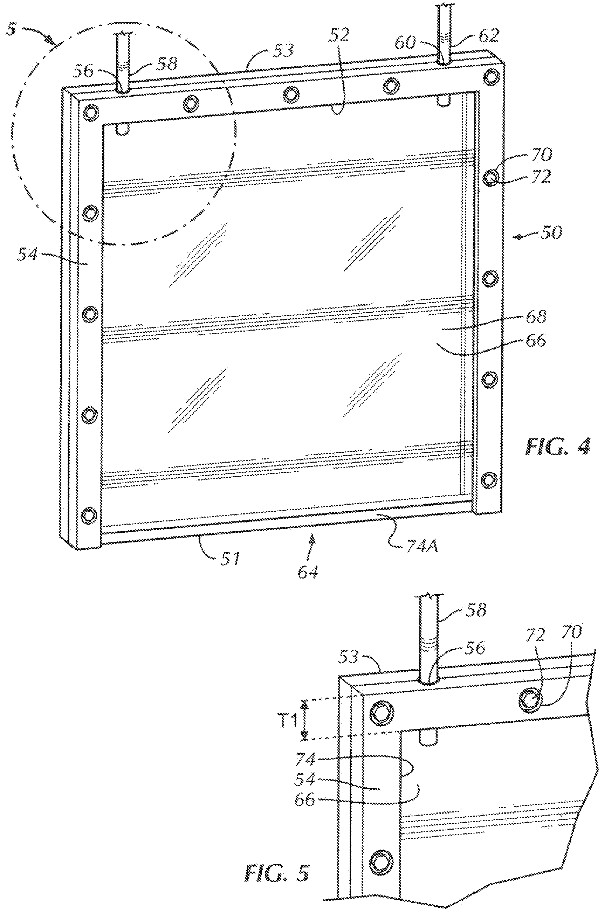

Figure 4 shows an example working fluid cassette 50 according to present principles. The cassette 50 is configured to fit snugly into the slot 34 and cassette frame receptacles R1, R2 defined between the cold plates 30, 32. Working fluid such as saline from a patient-engageable heat exchange member such as the catheter 12 or pad 18 flows through the cassette 50 in operation, with the working fluid exchanging heat with the refrigerant in the cold plates. In example embodiments, the cassette 50 is a low cost single-use disposable item that can contain, e.g., sterile saline which circulates through the catheter 12, The cassette may be placed by a medical caregiver in the slot 34 between the cold plates 30, 32 and the membrane portion which defines a space or working fluid chamber through which the example saline flows inflates when the working fluid flows through it, achieving thermal contact with the cold plates 30, 32.

In the example shown, the cassette 50 includes a frame 52 defining a periphery and a preferably rectilinear opening bounded as shown on at least three sides by the periphery of the frame. In the non-limiting example shown, the frame includes an elongated parallelepiped-shaped top rail 53 and elongated parallelepiped-shaped left and right side rads 54 parallel to each other and perpendicular to the top rail 32. The example frame 52 has no bottom rail opposite the top rail. In any case, the example frame 52 is rectilinear and is configured for being closely received between the two cold plates 30, 32, with the side rails 54 slidably engageable with the frame receptacles R1, R2 between the cold plates 30, 32 and with the below-described membrane assembly passed through the slot 36 to be in close juxtaposition with the refrigerant channels in the cold plates.

In cross-references to Figures 4 and 5, the frame, in the example shown, the top rail 53 thereof, is formed with a fluid inlet 56 in which an inlet tube 58 has been disposed and a fluid outlet 60 in which an outlet tube 62 has been disposed. Both the inlet and outlet establish respective fluid passageways through the frame into the opening. The inlet and outlet tubes 58, 62 may be engaged with the fluid return and supply lines L3, L4 that are associated with the catheter 12. The tubes 58, 62 may terminate at just below the top rail 53 (Figure 4), or they may extend any desired length down to the bottom of the assembly, i.e., the tubes 58, 62 may extend almost the entire length of the left and right side rails 54, ending just above the below-described bottom seam of the membrane assembly.

Indeed, a polymeric membrane assembly 64 is connected to the frame 52, blocking the opening that is bounded on three sides by the frame as shown. The membrane assembly includes a first membrane 66 that is parallel to and closely spaced from a second membrane 68, leaving a space there between which establishes a working fluid chamber. The fluid inlet 56 and fluid outlet 60 communicate with the space between the membranes 66, 68. At least one and preferably both of the membranes 66, 68 are disposed in tension in the opening. The space between the membranes is expandable when filled with working fluid.

In one example, each membrane is no more than two mils (0.002") (0.051 mm) thick and more preferably is between one mil and two mils in thickness (0.001" - 0.002") (25.4 - 50.8 micrometres), inclusive. The example preferred membranes 66, 68 are coextensive with the opening and like the opening are more or less square, with the length of top and bottom edges of the example membranes being approximately equal (within ±10% and more preferably within ±5%) of the lengths of the left and right edges of the membranes. Thus, the working fluid chamber between the membranes is also rectilinear and in the preferred embodiment no obstructions exist between the membranes, meaning the working fluid chamber is a complete rectilinear, more or less square chamber.

Owing to the thinness of the membranes 66, 68 and the closeness of the cold plates 30, 32 to each other and to the membrane assembly between them when the cassette is engaged with the cold plates, the system shown in the figures affords low impedance of heat transfer between the refrigerant circulating in the cold plates and the working fluid circulating between the membranes 66, 68. The working fluid chamber between the
membranes inflates due to backpressure generated by working fluid flow, eliminating or reducing the need for a moving mechanism in the cold plates. Moreover, the narrow slot 34 between the two cold plates provides better heat transfer by reducing the conductive path length between the cold plates and the working fluid, The frame allows for ease of handling, such as insertion and removal of the cassette with/from the cold plates.

With respect to the example working fluid chamber between the membranes 66, 68 having a width-to-length aspect ratio near 1:1 (i.e., square or nearly so), the amount of backpressure required to induce working fluid flow through heat exchanger is reduced compared to a less square configuration, This reduces the amount of work that a working fluid pump mast perform, which is desirable for two reasons. One, since the pump may be disposable, lower performance requirements translate into a lower cost disposable and quieter system. For instance, peristaltic roller pumps offer quiet operation and a low-cost disposable element, but operate most efficiently when only modest pressures are required. Two, lowering the working fluid pump work reduces the amount of heat transferred into the working fluid by the pump itself. Also, a low width/length aspect ratio results in slower working fluid velocity which reduces amount of mixing, but this otherwise desirable (from a heat exchange standpoint) effect is negligible in the present example system since the Reynolds numbers are typically < 1000, suggesting a laminar flow regime.

Furthermore, a low width/length aspect ratio significantly reduces the number of bends (or "corners") in the fluid How path. These bends are areas of mixing for the fluid which promotes heat transfer. Without them, a fluid boundary layer builds up. However, this effect is offset herein by maintaining a narrow slot between the cold plates. This way the primary heat transfer mechanism is by conduction, but the conduction path length (and therefore boundary layer) is small, resulting in a relatively high rate of heat transfer.

In preferred examples, the membranes 66, 68 are stretched under tension during assembly to the frame. This tension can be maintained over the shelf life of the product. Pretensioning minimizes wrinkles in material, which is beneficial because wrinkles can impede working fluid flow and create air gaps which reduce heat transfer between the working fluid and cold plates. Wrinkles can also complicate insertion of the membrane assembly into the narrow slot 34.

To establish pre-tensioning of the membranes" the frame may be made in halves and posts such as threaded fasteners 70 (Figure 5) can extend transversely to one half of the frame, with the membranes 66, 68 being stretched over the posts and boles made in the membranes to receive the posts. The other half of the frame is then positioned to sandwich a rectilinear border portion 74 (only the innermost portion of which is shown, in Figure 5) of the membrane assembly between the frame halves, and a closure such as respective nuts 72 engaged with the posts 70 to hold the frame halves together with the membrane assembly tension between the frame halves. Figure 4 shows that the working fluid chamber is closed off at the bottom by a bottom seam 74A of the membrane assembly, which is part of the border portion 74.

In the border portion 74, at least one and preferably more layers of polymer film may be used to reinforce the membranes 66, 68 to establish welded seams through which (at the sides of the membrane assembly) the post boles are formed, allowing for easier fabrication. By placing reinforcing layers on the border portion 74 only, the central "window" of the membrane assembly consists only of a single thin layer membrane 'between the working fluid and one of the cold plates 30, 32 to minimize impeding heat transfer. A die-cut reinforcement layer may be used which reinforces the entire perimeter with one piece of material.

In some examples, the polymer membranes 66, 68 are highly stretchable, at least greater than 25% elongation. This allows the membranes to change from the empty flat state shown in Figures 4 and 5 to an inflated shape (within the slot 34 between the cold plates) without wrinkling. It also allows the membranes to easily conform to features on the faces of the cold plates.

Additionally, the membranes may be made of a material which can also be made into tubing. Tubes such as the inlet and outlet tubes 58, 62 shown in Figure 4 can then be thermally welded (e.g., using RF sealing) to the membranes, which is more reliable and quicker than adhesive bonding. The membranes 66, 68 need not provide their own lateral support because the cold plates 32, 34 and frame provide the support for the inflated membrane assembly, allowing it to withstand the pressure generated as a result of working fluid flowing through between the membranes. Structural features may be located on the cold plates to optimize heat transfer. This can be economically advantageous because the cold plates are reusable components. Manifolds can be cut into the cold plates to even out the distribution of saline flow.

While the particular PATIENT HEAT EXCHANGE SYSTEM WITH TRANSPARENT WALL FOR ViEWING CIRCULATING REFRIGERANT is herein shown and described in detail, the scope of the present invention is to be limited by nothing other than the appended claims.

## Claims

1. A heat exchange system for exchanging heat with working fluid from an intravascular heat exchange catheter (12) or an external heat exchange pad (18), comprising:
a refrigerant circuit (38, 40, 42) configured for circulating refrigerant between a compressor and first and second cold plates (30, 32) between which a working fluid cassette (50) is disposable, at least a first one of the cold plates (32) having a transparent outer wall (32a) through which a person can view refrigerant flowing in the refrigerant circuit.

2. The system of claim 1, wherein the system is configured for circulating working fluid between the working fluid cassette (50) and the catheter (12).

3. The system of claim 1, wherein the system is configured for circulating working fluid between the working fluid cassette (50) and the pad (18).

4. The system of claim 1, wherein a cassette slot (34) is defined between the cold plates (30, 32) for receiving the working fluid cassette (50).

5. The system of claim 1, wherein a distance between the cold plates (30, 32) is less than forty mils (0.040") (1.02 millimetres).

6. The system of claim 1, comprising plural fasteners (44) holding the outer wall (32a) onto metal structure of the first cold plate (30).

7. The system of claim 6, wherein the fasteners (44) are arranged in four rows, one respective row along each of four edges of the outer wall (32a).

8. The system of claim 1, wherein the cold plates (30, 32) abut each other along left and right side walls (36).

9. The system of claim 8, wherein respective vertically elongated cassette frame receptacles (R1, R2) are located immediately inboard of the respective side walls (36) with the slot (34) extending between the side walls (36) and terminating at the receptacles (R1, R2), the frame receptacles (R1, R2) being wider than the slot (34).

10. The system of claim 1, wherein at least one cold plate (30, 32) is formed with a serpentine passageway (42) through which the refrigerant flows.

11. The system of claim 1 wherein the cold plates (30, 32) comprise two heat transfer plates parallel to each other and defining a slot (34) between them configured for receiving the working fluid cassette (50) through which working fluid flows to and from an intravascular catheter (12) in a working fluid circuit,
wherein the refrigerant circuit (38, 40, 42) is the only fluid circuit in thermal contact with the working fluid circuit other than a bloodstream of a patient in which the catheter (12) can be positioned.

12. A method of operating a heat exchange system exchanging heat with working fluid from an intravascular heat exchange catheter (12) or an external heat exchange pad (18), the method comprising:
circulating refrigerant between a compressor and first and second cold plates (30, 32) between which a working fluid cassette is disposable; and
enabling a person to view the refrigerant through a transparent outer wall of one of the cold plates as it circulates.

13. The method of Claim 12, comprising:
circulating working fluid between an intravascular heat exchange catheter (12) and a fluid cassette (50) disposed in contact with the cold plate (32) to exchange heat between the refrigerant and the working fluid through the cold plate (32).

14. The method of Claim 12, wherein the cold plate (32) is a first cold plate and the method comprises circulating working fluid between the first cold plate (32) and a second cold plate (30), at least one of the cold plates (30, 32) being at least partially transparent.

## Patentansprüche

1. Wärmeaustauschsystem zum Austausch von Wärme mit Arbeitsfluid von einem intravaskulären Wärmeaustauschkatheter (12) oder einem externen Wärmeaustauschkissen (18), umfassend:
einen Kältemittelkreislauf (38, 40, 42), ausgelegt zum Umwälzen von Kältemittel zwischen einem Kompressor und einer ersten und einer zweiten Kälteplatte (30, 32), zwischen denen eine Arbeitsfluidkassette (50) angeordnet werden kann, wobei mindestens eine der Kälteplatten (32) eine transparente Außenwand (32a) aufweist, durch die eine Person ein Kältemittel beobachten kann, das in dem Kältemittelkreislauf fließt.

2. System nach Anspruch 1, wobei das System zum Umwälzen von Arbeitsfluid zwischen der Arbeitsfluidkassette (50) und dem Katheter (12) ausgelegt ist.

3. System nach Anspruch 1, wobei das System zum Umwälzen von Arbeitsfluid zwischen der Arbeitsfluidkassette (50) und dem Kissen (18) ausgelegt ist.

4. System nach Anspruch 1, wobei ein Kassettenschlitz (34) zur Aufnahme der Arbeitsfluidkassette (50) zwischen den Kälteplatten (30, 32) begrenzt ist.

5. System nach Anspruch 1, wobei ein Abstand zwischen den Kälteplatten (30, 32) weniger als vierzig Mil (0,040") (1,02 mm) beträgt.

6. System nach Anspruch 1, das mehrere Befestigungselemente (44) umfasst, die die Außenwand (32a) auf der Metallstruktur der ersten Kälteplatte (30) halten.

7. System nach Anspruch 6, wobei die Befestigungselemente (44) in vier Reihen angeordnet sind, jeweils eine Reihe entlang jeder von vier Kanten der Außenwand (32a).

8. System nach Anspruch 1, wobei die Kälteplatten (30, 32) entlang der linken und rechten Seitenwand (36) aneinander anliegen.

9. System nach Anspruch 8, wobei jeweils vertikal verlängerte Kassettenrahmenaufnehmer (R1, R2) unmittelbar innerhalb der jeweiligen Seitenwände (36) angeordnet sind, wobei sich der Schlitz (34) zwischen den Seitenwänden (36) und an den Aufnehmern (R1, R2) endend erstreckt, wobei die Rahmenaufnehmer (R1, R2) breiter als der Schlitz (34) sind.

10. System nach Anspruch 1, wobei mindestens eine Kälteplatte (30, 32) mit einer Schlangenleitung (42) ausgebildet ist, durch die das Kältemittel fließt.

11. System nach Anspruch 1, wobei die Kälteplatten (30, 32) zwei Wärmeübertragungsplatten umfassen, die parallel zueinander angeordnet sind und zwischen sich einen Schlitz (34) begrenzen, der zur Aufnahme der Arbeitsfluidkassette (50) ausgelegt ist, durch die das Arbeitsfluid in einem Arbeitsfluidkreislauf zu und von einem intravaskulären Katheter (12) fließt, wobei der Kältemittelkreislauf (38, 40, 42) der einzige Fluidkreislauf ist, der in thermischem Kontakt mit dem Arbeitsfluidkreis steht, abgesehen von einem Blutstrom eines Patienten, in dem der Katheter (12) positioniert werden kann.

12. Verfahren zum Betrieb eines Wärmeaustauschsystems, das Wärme mit Arbeitsfluid von einem intravaskulären Wärmeaustauschkatheter (12) oder einem externen Wärmeaustauschkissen (18) tauscht, wobei das Verfahren umfasst:
Umwälzen von Kältemittel zwischen einem Kompressor und einer ersten und zweiten Kälteplatte (30, 32), zwischen denen eine Arbeitsfluidkassette eingebracht werden kann; und
Versetzen einer Person in die Lage, das Kältemittel durch eine transparente Außenwand einer der Kälteplatten zu beobachten, während es umgewälzt wird.

13. Verfahren nach Anspruch 12, umfassend:
Umwälzen von Arbeitsfluid zwischen einem intravaskulären Wärmeaustauschkatheter (12) und einer Fluidkassette (50), die in Kontakt mit der Kälteplatte (32) angeordnet ist, um über die Kälteplatte (32) Wärme zwischen dem Kältemittel und dem Arbeitsfluid auszutauschen.

14. Verfahren nach Anspruch 12, wobei die Kälteplatte (32) eine erste Kälteplatte ist und das Verfahren das Umwälzen von Arbeitsfluid zwischen der ersten Kälteplatte (32) und einer zweiten Kälteplatte (30) umfasst, wobei mindestens eine der Kälteplatten (30, 32) zumindest teilweise transparent ist.

## Revendications

1. Système d'échange de chaleur destiné à l'échange de chaleur avec un fluide de travail provenant d'un cathéter intravasculaire d'échange de chaleur (12) ou d'un coussinet externe d'échange de chaleur (18), comprenant :
un circuit de réfrigération (38, 40, 42), conçu pour la mise en circulation d'un réfrigérant entre un compresseur et des première et seconde plaques froides (30, 32) entre lesquelles on peut disposer une cassette de fluide de travail (50), au moins une des plaques froides (32) comportant une paroi externe transparente (32a) à travers laquelle une personne peut visualiser le réfrigérant s'écouler dans le circuit de réfrigération.

2. Système selon la revendication 1, dans lequel le système est conçu pour la mise en circulation d'un fluide de travail, entre la cassette de fluide de travail (50) et le cathéter (12).

3. Système selon la revendication 1, dans lequel le système est conçu pour la mise en circulation d'un fluide de travail, entre la cassette de fluide de travail (50) et le coussinet (18).

4. Système selon la revendication 1, dans lequel une fente pour cassette (34) est définie entre les plaques froides (30, 32) destinées à la réception de la cassette de fluide de travail (50).

5. Système selon la revendication 1, dans lequel une distance entre les plaques froides (30, 32) est inférieure à 40 millièmes de pouce (0,040") (1,02 mm).

6. Système selon la revendication 1, comprenant plusieurs éléments de fixation (44) qui maintiennent la paroi externe (32a) sur une structure métallique de la première plaque froide (30).

7. Système selon la revendication 6, dans lequel les éléments de fixation (44) sont agencés en quatre rangées, une rangée respective le long de chacun des quatre bords de la paroi externe (32a).

8. Système selon la revendication 1, dans lequel les plaques froides (30, 32) viennent buter les unes contre les autres le long des parois latérales gauche et droite (36).

9. Système selon la revendication 8, dans lequel les réceptacles respectifs de cadre de cassette allongée verticalement (R1, R2) sont situés immédiatement à l'intérieur des parois latérales (36) respectives avec la fente (34) s'étendant entre les parois latérales (36) et se terminant au niveau des réceptacles (R1, R2), les réceptacles de cadre (R1, R2) étant plus larges que la fente (34).

10. Système selon la revendication 1, dans lequel au moins une plaque froide (30, 32) est pourvue d'une voie de passage en serpentin (42) à travers laquelle s'écoule le réfrigérant.

11. Système selon la revendication 1, dans lequel les plaques froides (30, 32) comprennent deux plaques parallèles de transfert de chaleur et définissant une fente (34) entre elles, conçues pour recevoir la cassette de fluide de travail (50) à travers laquelle un fluide de travail s'écoule vers et depuis un cathéter intravasculaire (12) dans un circuit de fluide de travail, le circuit réfrigérant (38, 40, 42) étant le seul circuit de fluide en contact thermique avec le circuit de fluide de travail autre qu'un flux sanguin d'un patient dans lequel peut être positionné le cathéter (12).

12. Procédé de fonctionnement d'un système d'échange de chaleur échangeant de la chaleur avec un fluide de travail provenant d'un cathéter intravasculaire d'échange de chaleur (12) ou d'un coussinet externe d'échange de chaleur (18), le procédé consistant à :
mettre en circulation un réfrigérant entre un compresseur et des première et seconde plaques froides (30, 32) entre lesquelles on peut disposer une cassette de fluide de travail ; et
permettre à une personne de visualiser le réfrigérant à travers une paroi externe transparente d'une des plaques froides pendant qu'il circule.

13. Procédé selon la revendication 12, comprenant :
la mise en circulation d'un fluide travail entre un cathéter intravasculaire d'échange de chaleur (12) et une cassette de fluide (50) disposée en contact avec la plaque froide (32) afin d'échanger de la chaleur entre le réfrigérant et le fluide travail à travers la plaque froide (32).

14. Procédé selon la revendication 12, dans lequel la plaque froide (32) est une première plaque froide et le procédé comprenant la mise en circulation d'un fluide travail entre la première plaque froide (32) et une seconde plaque froide (30), au moins une des plaques froides (30, 32) étant au moins partiellement transparente.
